# EUROPEAN PATENT APPLICATION

(11) **EP 1 712 233 A1**
(43) Date of publication of application: **18.10.2006**
(21) Application number: 05710140.4
(22) Date of filing: 04.02.2005
(51) Int. Cl.: A61K 31/4745, A61K 9/08, A61K 47/16

(54) **AQUEOUS SOLUTION CONTAINING PYRAZOLOACRIDONE DERIVATIVE**

(30) Priority: 06.02.2004 JP 2004030790
(71) Applicant: KYOWA HAKKO KOGYO CO., LTD., Chiyoda-ku, Tokyo 100-8185 (JP)
(72) Inventor: UENO, Yuji c/o Pharmaceutical Research Center, Nagaizumi-cho,Sunto-gun,Shizuoka411-8731 (JP); UENO, Yasuhiko c/o Fuji Plant, Nagaizumi-cho,Sunto-gun,Shizuoka411-8731 (JP)
(74) Representative: Vossius & Partner
(86) International application number: PCT/JP2005/002088
(87) International publication number: WO 2005/074936

(57) **Abstract**

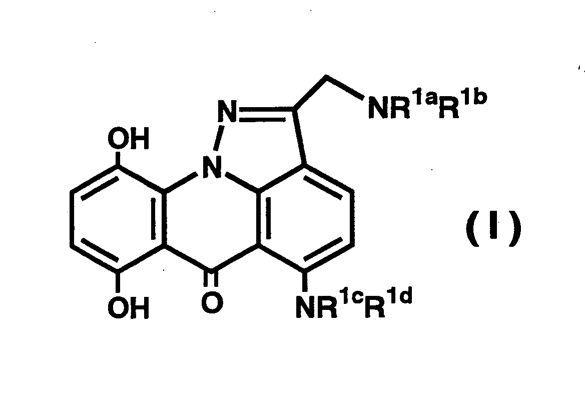

[wherein R^{1a}, R^{1b}, R^{1c}, and R^{1d} may be the same or different and each represent a hydrogen atom, lower alkyl, -(CH₂)ₚ-X (wherein p represents an integer of 1 to 6, X represents hydroxy, lower alkoxy, or -NR^{2a}R^{2b}), or -CH[(CH₂)ₙOH]₂ (wherein n represents an integer of 1 to 5)]

The present invention provides an aqueous solution having a pH of 3.5 or less and containing a pyrazoloacrydone derivative or a pharmaceutically acceptable salt thereof represented by, for example, above general formula (I).

## Description

### Technical Field

The present invention relates to an aqueous solution which comprises pyrazoloacrydone derivatives or a pharmaceutically acceptable salt thereof.

### Background Art

Pyrazoloacrydone derivatives are known to have DNA intercalation activity and to show anti-tumor effect [for example, J. Med. Chem., vol. 37, pp. 1028-1032, (1994), and Japanese Published Unexamined Patent Application No.1064/1993].

The pyrazoloacrydone derivatives are known to be degraded oxidatively in aqueous solutions easily. As a stabilizing method for an aqueous solution comprising a pyrazoloacrydone derivative or a pharmaceutically acceptable salt thereof and a drug product in which the solution is filled into a drug container, there has been known a method of adding an acid to the aqueous solution containing the pyrazoloacrydone derivative or pharmaceutically acceptable salt thereof and replacing the air in a closed container (drug container) comprising the aqueous solution with an inert gas (WO 00/21962). In this method, the concentration of the inert gas in the container is preferably 90% (vol/vol) or more, more preferably 95% (vol/vol) or more, furthermore preferably 99% (vol/vol) or more. The replacement of the air in a drug container with an inert gas is a common technique. However, in order to achieve such a high replacement ratio of the air with inert-gas as described above, a highly accurate method or technique of performing gas replacement is required. In addition, the integrity of the drug container is important. For example, when a common combination of a glass vial and a rubber stopper is used, it is impossible to avoid the influx of a trace amount of the outside air (air and so on) into the glass vial. In other words, for long term storage, a advanced technique or a method is necessary for maintaining the high replacement ratio of the air in a drug container with inert-gas. Accordingly, it is desired an aqueous solution comprising the pyrazoloacrydone derivative or a pharmaceutically acceptable salt thereof and a drug product in which the solution is filled into a drug container can be prepared by a simple operation and well stored for a long period of time.

### Disclosure of the Invention

An object of the present invention is to provide an aqueous solution and a drug which comprises for example a pyrazoloacrydone derivative or a pharmaceutically acceptable salt thereof and which can be formulated by a simple operation and have excellent stability.

The present invention relates to the following aspects (1) to (7):
(1) An aqueous solution having a pH of 3.5 or less and comprising a pyrazoloacrydone derivative represented by general formula (I): 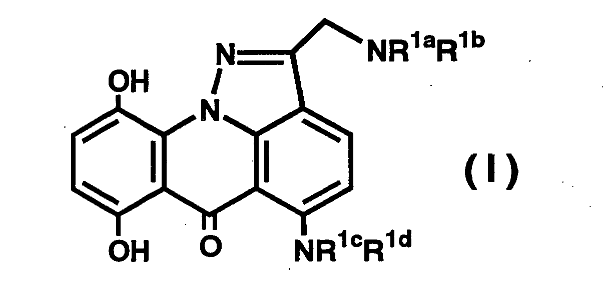 [wherein R^{1a}, R^{1b}, R^{1c}, and R^{1d} may be the same or different and each represents a hydrogen atom, lower alkyl, -(CH₂)ₚ-X <wherein p represents an integer of 1 to 6, X represents hydroxy, lower alkoxy, or -NR^{2a}R^{2b} {wherein R^{2a} and R^{2b} may be the same or different and each represents a hydrogen atom, lower alkyl, or -(CH₂)ₘ-Y [wherein m represents an integer of 1 to 6, Y represents hydroxyl, lower alkoxy, or -NR^{3a}R^{3b} (wherein R^{3a} and R^{3b} may be the same or different and each represents a hydrogen atom or lower alkyl)], or R^{2a} and R^{2b} are combined together with the adjacent nitrogen atom to form a heterocyclic group }>, or -CH[(CH₂)ₙOH]₂ (wherein n represents an integer of 1 to 5)] or a pharmaceutically acceptable salt thereof.
(2) The aqueous solution according to (1), wherein the pH is 2 to 3;
(3) The aqueous solution according to (1) or (2), the solution which comprising edetic acid or a salt thereof;
(4) The aqueous solution according to (3), wherein the content of the edetic acid or the salt thereof is 0.01 to 0.20 weight parts per 1 weight part of the pyrazoloacrydone derivative represented by the general formula (I) or the pharmaceutically acceptable salt thereof.
(5) A drug product in which the solution according to any one of (1) to (4) is filled into a drug container;
(6) A method for stabilizing a pyrazoloacrydone derivative represented by general formula (I): 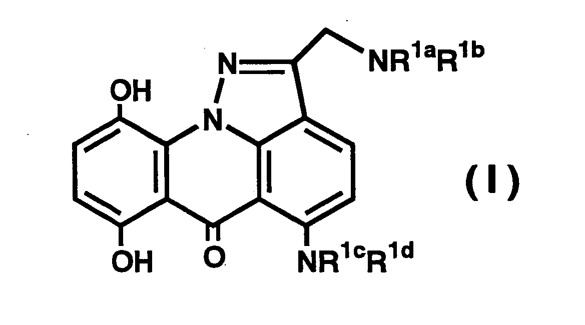 (wherein R^{1a}, R^{1b}, R^{1c}, and R^{1d} have the same meanings as defined above, respectively) or a pharmaceutically acceptable salt thereof in an aqueous solution by adjusting the pH of the aqueous solution comprising the pyrazoloacrydone derivative or the pharmaceutically acceptable salt thereof to 3.5 or less.
(7) The method according to (6), wherein the pH is adjusted to 2 to 3.

The aqueous solution of the present invention and the drug product of the present invention in which the solution is filled into a drug container can be used for example, as an injection in treating malignant tumor. And when used, they may be properly diluted with commonly used transfusion fluids, such as a physiological saline solution and a sugar solution, if necessary.

In the definition of the general formula (I), examples of the lower alkyl and the alkyl moiety of the lower alkoxy include linear or branched alkyls having 1 to 6 carbon atoms, such as, methyl, ethyl, propyl, isopropyl, butyl, isobutyl, sec-butyl, tert-butyl, pentyl, and hexyl.

Examples of the heterocyclic group formed together with the adjacent nitrogen atom include pyrrolidinyl, piperidino, piperazinyl, morpholino, thiomorpholino, quinolyl, pyrimidinyl, pyridazinyl, pyridyl, pyrrolyl, imidazolyl, pyrazolyl and the like. Among them, pyrrolidinyl, piperidino, piperazinyl, or morpholino are preferable.

Hereinafter, compounds represented by the general formula (I) are referred to as compound (I).

Examples of the pharmaceutically acceptable salts of the compound (I) include inorganic acid salts such as hydrochloride, hydrobromide, sulfate, and phosphate; and organic acid salts such as acetate, oxalate, malonate, maleate, fumarate, tartrate, succinate, and citrate.

The compound (I) is a known compound as disclosed in Japanese Published Unexamined Patent Application No. 1064/1993, and can be produced by, for example, the production method disclosed in Japanese Published Unexamined Patent Application No. 1064/1993.

Examples of compound (I) include the compounds shown in below Table 1.

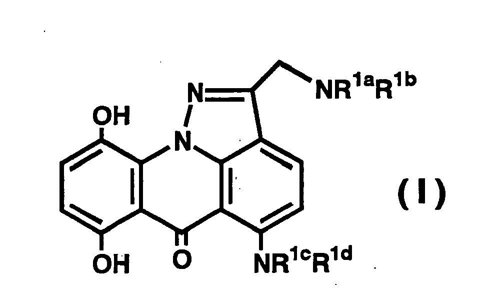

**Table 1**

| Compound No. | NR^{1a}R^{1b} | NR^{1c}R^{1d} |
|---|---|---|
| 1 | NH (CH₂) ₂NH₂ | NH (CH₂) ₂NH₂ |
| 2 | N(C₂H₅)₂ | NH (CH₂) ₂NH₂ |
| 3 | N(C₂H₅)₂ | NH(CH₂)₂N(CH₃)₂ |
| 4 | NH(CH₂)₂OH | NH(CH₂)₂NH₂ |
| 5 | NH(CH₂)₂OH | NH (CH₂) ₃NH₂ |
| 6 | NH(CH₂)₂OH | NH(CH₂)₂NH(CH₂)₂OH |
| 7 | NH(CH₂)₂OH | NH (CH₂) ₂NHCH₃ |
| 8 | NH (CH₂) ₂OH | NH(CH₂)₂N(CH₃)₂ |
| 9 | N[(CH₂)₂OH]₂ | NH(CH₂)₂N(CH₃)₂ |
| 10 | NHCH(CH₂OH)₂ | NH (CH₂)₂NH₂ |
| 11 | NH(CH₂)₂OCH₃ | NH (CH₂)₂NH₂ |
| 12 | NHCH(CH₂OH)₂ | NH(CH₂)₂NH(CH₂)₂OH |
| 13 | NHCH(CH₂OH)₂ | NH (CH₂)₃NH₂ |
| 14 | NHCH(CH₂OH)₂ | 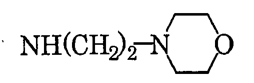 |

The aqueous solution of the present invention can be filled into a drug container such as an ampule, vial, and syringe to prepare the drug product of the present invention. The material and shape of the drug container are not specifically limited. Examples of the material of the drug container include glass, resin, and the like. Examples of the resins include polyethylene, polystylene, polycarbonate, polypropylene, polyvinyl chloride, nylon-6, polyethylene terephthalate, polyvinyl alcohol, polyacrylonitrile, polyvinylidene chloride, and the like.

It is not necessary to replace the air present in the drug container with an inert gas such as argon gas, nitrogen gas, helium gas, and carbon dioxide gas. However, the air in the drug container may be properly replaced with an inert gas if necessary. The replacement with an inert gas can be performed according to an ordinary method. For example, the aqueous solution may be filled into the drug container under an inert gas atmosphere, or an inert gas may be injected into the drug container after the internal pressure of the drug container is reduced.

The pH of the aqueous solution of the present invention may be equal to or less than 3.5, but preferably between 1 and 3.5, more preferably between 2 and 3.5, most preferably between 2 and 3. The pH of the aqueous solution of the present invention may be adjusted with an alkali such as sodium hydroxide, potassium hydroxide, or potassium dihydrogen phosphate; or with an acid such as citric acid, tartaric acid, lactic acid, acetic acid, hydrochloric acid, or sulfuric acid. These alkali and acid may be used alone or in combination thereof for adjusting the pH of the aqueous solution to a desired value.

The aqueous solution of the present invention comprises the compound (I) or a pharmaceutically acceptable salt thereof preferably at a concentration of 0.1 to 100 mg/mL, more preferably at 0.5 to 50 mg/mL, most preferably at 1 to 10 mg/mL.

The aqueous solution of the present invention may comprise edetic acid or an edetate such as sodium edetate or calcium disodium edetate. The aqueous solution of the present invention can be further stabilized by the presence of edetic acid or edetate preferably at 0.001 to 0.50 weight parts, more preferably at 0.005 to 0.30 weight parts, further preferably at 0.007 to 0.25 weight parts, most preferably at 0.01 to 0.20 weight parts per 1 weight part of the compound (I) or a pharmaceutically acceptable salt thereof.

The aqueous solution of the present invention may further comprise, beside above, an excipient which is commonly used in drug formulations, for example, a tonicity agent, a soothing agent, an antioxidant, or an anti-adsorption agent or the like.

Examples of the tonicity agent include sodium chloride, glucose, fructose, mannitol, xylitol, glycerin, propylene glycol, benzyl alcohol and the like.

Examples of the soothing agent include inositol, chlorobutanol, propylene glycol, benzyl alcohol and the like.

Examples of the antioxidant include ascorbic acid, tocopherol, cysteine hydrochloride, sodium thioglycolate and the like.

Examples of the anti-adsorption agent include polyoxyethylene sorbitan fatty acid esters such as polyoxyethylene sorbitan monooleate and polyoxyethylene sorbitan monostearate, sorbitan fatty acid esters such as sorbitan monolaurate and sorbitan monoparmitate, and polyethylene glycol fatty acid ethers such as polyoxyethylene lauryl ether and the like.

Example of the preparation method of the aqueous solution and drugs of the present invention will be shown below.

### <Preparation method>

The aqueous solution of the present invention can be obtained by dissolving the compound (I) and an acid, and, if necessary an excipient, in distilled water for injection or the like and then adjusting the pH of the resulting aqueous solution to 3.5 or less with a proper amount of an acid and/or an alkali, if necessary. When the aqueous solution of the present invention comprises edetic acid or an edetate such as sodium edetate and calcium disodium edetate, the edetic acid or the edetate may be, for example, added to the above-mentioned aqueous solution the pH of which is adjusted to 3.5 or less, and then the pH of the resulting aqueous solution may be adjusted to 3.5 or less. Alternatively, edetic acid or the edetate may be dissolved in distilled water for injection together with the compound (I) or the like, and then the pH of the resulting aqueous solution may be adjusted by the same method as above.

In addition, the drug product of the present invention can be obtained by, for example, subjecting the aqueous solution of the present invention prepared above to sterile filtration using a membrane filter and the like and then filling the solution into a drug container. Here, the air present in the drug container may be replaced with an inert gas, if necessary.

The stabilizing method of the present invention can be performed by a similar method to the above-mentioned method for preparing the aqueous solution of the present invention. In the stabilizing method of the present invention, the pH of the aqueous solution, the content of the compound (I) or a pharmaceutically acceptable salt thereof in the aqueous solution, the addition of edetic acid, an edentate or the like, and conditions for other excipient and the like may be the same as those shown above in the preparation of the aqueous solution of the present invention.

The effect of the present invention will now be described with reference to Test Examples, but the present invention is not limited to them.

### Test Example 1: Stability of compound (I)

The drug products 1 to 4 and drug products 10 to 12 (drug container: polypropylene tube) obtained in Examples 1 to 4 and in Comparative Examples 1 to 3, respectively, were stored in the atmosphere at 40°C for 1 month. The pH of the aqueous solution and the production amount of related substances (compounds other than compound 5, which comprise degradation products of compound 5 and which exist in the aqueous solution) in the aqueous solution were measured at the beginning of the test and after the storage.

The production amount of the related substances was measured by high-performance liquid chromatography (HPLC). The conditions for the measurement were as follows:

### <HPLC conditions>

### Mobile phase:

A-solution; 0.1 mol/L phosphate buffer (pH 2.5): acetonitrile = 9 : 1
B-solution; 0.1 mol/L phosphate buffer (pH 2.5): acetonitrile = 1 : 1

### Gradient conditions:

| Time (min) | Mixture ratio of mobile phase (A-solution/B-solution) |
|---|---|
| 0 | 90/10 |
| 21 | 82/18 |
| 40 | 0/100 |
| 50 | 0/100 |

Column: YMC-Pack (ODS-AM, AM-312, TSK SP-5PW 150 x 6.0 mm I.D.)
Flow rate: 1.3 mL/min
Column temperature: 40°C
Sample temperature: 25°C
Detection wavelength: 400 nm

The results are shown in Table 2. The production amount of the related substances is shown as an increment of the related substances during the storage by calculating a difference in the amount of the related substances after the strage with the amount at the beginning of the test.

**Table 2**

| | pH of aqueous solution (at the beginning of test) | pH of aqueous solution (after 1 month at 40°C) | Increment of related substances (%) |
|---|---|---|---|
| Drug product 1 | 2.0 | 2.0 | 0.3 |
| Drug product 2 | 2.5 | 2.5 | 0.5 |
| Drug product 3 | 3.0 | 3.0 | 1.0 |
| Drug product 4 | 3.5 | 3.5 | 1.6 |
| Drug product 10 | 3.9 | 3.8 | 2.3 |
| Drug product 11 | 4.5 | 4.2 | 5.8 |
| Drug product 12 | 5.0 | 4.4 | 7.3 |

As shown in Table 2, when the drug product is charged with an aqueous solution having a pH of 3.5 or less, the increment of the related substances was significantly suppressed. In other words, the increment of the related substances in an aqueous solution containing compound 5 can be suppressed by adjusting the pH of the solution to 3.5 or less.

### Test Example 2: Effect of adding edetate on stability to compound (I)

The drug products 5 to 9 (drug container: glass vial) obtained in Examples 5 to 9, respectively, were stored in the atmosphere at 40°C for 1 month. The pH of the aqueous solution and the production amount of related substances (compounds other than compound 5, which comprise degradation products of compound 5 and which exist in the aqueous solution) in the aqueous solution were measured at the beginning of the test and after the storage. The production amount of the related substances was measured at the same HPLC conditions as those in Test Example 1.

The result are shown in Table 3. The production amount of the related substances is shown as an increment of the related substances during the storage by calculating a difference of the amount of the related substances after the strage with the amount at the beginning of the test.

**Table 3**

| | pH of aqueous solution (at the beginning of test) | pH of aqueous solution (after 1 month at 40' C) | increment of related substances (%) |
|---|---|---|---|
| Drug product 5 | 2.5 | 2.5 | 0.5 |
| Drug product 6 | 2.5 | 2.5 | 0.3 |
| Drug product 7 | 2.5 | 2.5 | 0.2 |
| Drug product 8 | 2.5 | 2.5 | 0.3 |
| Drug product 9 | 2.5 | 2.5 | 0.3 |

It was confirmed that the aqueous solution of the present invention was also stable in the glass vial too and that the pH of the aqueous solution did not change even if the solution was stored at 40°C for 1 month. In addition, the production of the related materials was further suppressed by the addition of disodium edetate (drug products 6 to 9).

From the results shown above, it was confirmed that the excellent preservation stability of the drug could be maintained by adjusting the pH of the aqueous solution to 3.5 or less and further maintained by adding an edetate such as disodium edetate to the solution, in a drug formulated by filling a drug in which an aqueous solution comprising compound (I) or pharmaceutically acceptable salt thereof is filled into a drug container, even if the air present in the drug container was not replaced with an inert gas.

### Best Mode for Carrying Out the Invention

The present invention will now be specifically described with reference to the following Examples, but those Examples are exemplary given for illustrating an embodiment of the present invention, and the present invention is not limited to them.

### Example 1: Aqueous solution 1 and drug product 1

Compound 5 dihydrochloride and lactic acid were dissolved in water for injection at 5 mg/mL and 0.9 mg/mL, respectively. The pH of the resulting aqueous solution was adjusted to 2.0 using a small amount of hydrochloric acid and sodium hydroxide solution to obtain aqueous solution 1. The resulting aqueous solution 1 was filled into a polypropylene tube to obtain drug product 1. Here, there was no replacement with an inert gas.

### Example 2: Aqueous solution 2 and drug product 2

Compound 5 dihydrochloride and lactic acid were dissolved in water for injection at 5 mg/mL and 0.9 mg/mL, respectively. The pH of the resulting aqueous solution was adjusted to 2.5 using a small amount of hydrochloric acid and sodium hydroxide solution to obtain aqueous solution 2. The resulting aqueous solution 2 was filled into a polypropylene tube to obtain drug product 2. Here, there was no replacement with an inert gas.

### Example 3: Aqueous solution 3 and drug product 3

Compound 5 dihydrochloride and lactic acid were dissolved in water for injection at 5 mg/mL and 0.9 mg/mL, respectively. The pH of the resulting aqueous solution was adjusted to 3.0 using a small amount of hydrochloric acid and sodium hydroxide solution to obtain aqueous solution 3. The resulting aqueous solution 3 was filled into a polypropylene tube to obtain drug product 3. Here, there was no replacement with an inert gas.

### Example 4: Aqueous solution 4 and drug product 4

Compound 5 dihydrochloride and lactic acid were dissolved in water for injection at 5 mg/mL and 0.9 mg/mL, respectively. The pH of the resulting aqueous solution was adjusted to 3.5 using a small amount of hydrochloric acid and sodium hydroxide solution to obtain aqueous solution 4. The resulting aqueous solution 4 was filled into a polypropylene tube to obtain drug product 4. Here, there was no replacement with an inert gas.

### Example 5: Aqueous solution 5 and drug product 5

Compound 5 dihydrochloride (0.55 g) was dissolved in water for injection(50mL). The pH of the resulting aqueous solution was adjusted to 2.5 using a small amount of hydrochloric acid. The total mass of the solution was adjusted to 55 g by adding distilled water for injection to obtain a bulk solution of compound 5 dihydrochloride. The bulk solution (10 mL) of compound 5 dihydrochloride was diluted with water (10 mL) for injection. The pH of the resulting aqueous solution was adjusted to 2.5 with a small amount of hydrochloric acid to obtain aqueous solution 5. The resulting aqueous solution 5 was subjected to sterile filtration in a clean bench, and then filled into glass vials at 1 mL/vial. The glass vials were each sealed with a rubber stopper and an aluminum cap to obtain drug product 5 (the concentration of compound 5 dihydrochloride: 5 mg/mL). Here, there was no replacement with an inert gas.

### Example 6: Aqueous solution 6 and drug product 6

Disodium edetate dihydrate (221 mg) was dissolved in water for injection so that the total amount was 100 mL (EDTA solution). The bulk solution (10 mL) of compound 5 dihydrochloride prepared in Example 5, the EDTA solution (0.5 mL), and distilled water (9.5 mL) for injection were mixed. The pH of the resulting aqueous solution was adjusted to 2.5 using a small amount of hydrochloric acid to obtain aqueous solution 6. The resulting aqueous solution 6 was subjected to sterile filtration in a clean bench, and then filled into glass vials at 1 mL/vial. The glass vials were each sealed with a rubber stopper and an aluminum cap to obtain drug product 6 (the concentration of dihydrochloride in compound 5: 5 mg/mL, the concentration of disodium edetate: 0.05 mg/mL). Here, there was no replacement with an inert gas.

### Example 7: Aqueous solution 7 and drug product 7

The bulk solution (10 mL) of compound 5 dihydrochloride prepared in Example 5, the EDTA solution (2.5 mL) prepared in Example 6, and distilled water (7.5 mL) for injection were mixed. The pH of the resulting aqueous solution was adjusted to 2.5 with a small amount of hydrochloric acid to obtain aqueous solution 7. The resulting aqueous solution 7 was subjected to sterile filtration in a clean bench, and then filled into glass vials at 1 mL/vial. The glass vials were each sealed with a rubber stopper and an aluminum cap to obtain drug product 7 (the concentration of compound 5 dihydrochloride: 5 mg/mL, the concentration of disodium edetate: 0.25 mg/mL). Here, there was no replacement with an inert gas.

### Example 8: Aqueous solution 8 and drug product 8

The undiluted solution (10 mL) of compound 5 dihydrochloride prepared in Example 5, the EDTA solution (5 mL) prepared in Example 6, and distilled water (5 mL) for injection were mixed. The pH of the resulting aqueous solution was adjusted to 2.5 using a small amount of hydrochloric acid to obtain aqueous solution 8. The resulting aqueous solution 8 was subjected to sterile filtration in a clean bench, and then filled into glass vials at 1 mL/vial. The glass vials were each sealed with a rubber stopper and an aluminum cap to obtain drug product 8 (the content of compound 5 dihydrochloride: 5 mg/mL, the content of disodium edetate: 0.5 mg/mL). Here, there was no replacement with an inert gas.

### Example 9: Aqueous solution 9 and drug product 9

The bulk solution (10 mL) of compound 5 dihydrochloride prepared in Example 5 and the EDTA solution (10 mL) prepared in Example 6 were mixed. The pH of the resulting aqueous solution was adjusted to 2.5 using a small amount of hydrochloric acid to obtain aqueous solution 9. The resulting aqueous solution 9 was subjected to sterile filtration in a clean bench, and then filled into glass vials at 1 mL/vial. The glass vials were each sealed with a rubber stoppre and an aluminum cap to obtain drug product 9 (the cocentration of compound 5 dihydrochloride: 5 mg/mL, the concentration of edetate: 1 mg/mL). Here, there was no replacement with an inert gas.

### Comparative Example 1: Aqueous solution 10 and drug product 10

Compound 5 Dihydrochloride and lactic acid were dissolved in water for injection at 5 mg/mL and 0.9 mg/mL, respectively. The pH of the resulting aqueous solution was adjusted to 3.9 using a small amount of hydrochloric acid and sodium hydroxide solution to obtain aqueous solution 10. The resulting aqueous solution 10 was filled into a polypropylene tube to obtain drug product 10. Here, there was no replacement with an inert gas.

### Comparative Example 2: Aqueous solution 11 and drug product 11

Compound 5 dihydrochloride and lactic acid in were dissolved in water for injection at 5 mg/mL and 0.9 mg/mL, respectively. The pH of the resulting aqueous solution was adjusted to 4.5 using a small amount of hydrochloric acid and sodium hydroxide solution to obtain aqueous solution 11. The resulting aqueous solution 11 was filled into a polypropylene tube to obtain drug product 11. Here, there was no replacement with an inert gas.

### Comparative Example 3: Aqueous solution 12 and drug product 12

Dihydrochloride and lactic acid in compound 5were dissolved in water for injection at 5 mg/mL and 0.9 mg/mL, respectively. The pH of the resulting aqueous solution was adjusted to 5.0 using a small amount of hydrochloric acid and sodium hydroxide solution to obtain aqueous solution 12. The resulting aqueous solution 12 was filled into a polypropylene tube to obtain drug product 12. Here, there was no replacement with an inert gas.

### Industrial Applicability

The present invention provides an aqueous solution and a drug product which comprises, for example, a pyrazoloacrydone derivative or a pharmaceutically acceptable salt thereof, and which can be formulated by a simple operation and have an excellent preservation stability.

## Claims

1. An aqueous solution having a pH of 3.5 or less and comprising a pyrazoloacrydone derivative represented by general formula (I): 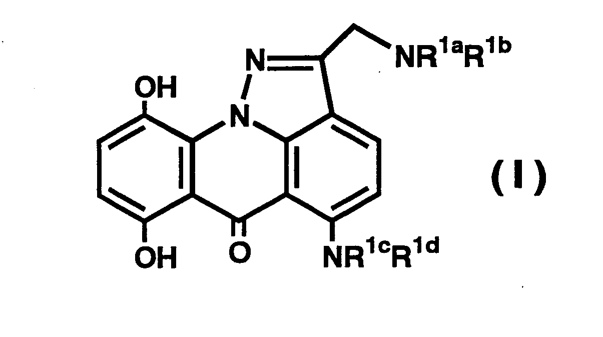 [wherein R^{1a}, R^{1b}, R^{1c}, and R^{1d} may be the same or different and each represents a hydrogen atom, lower alkyl, -(CH₂)ₚ-X <wherein p represents an integer of 1 to 6, X represents hydroxyl, lower alkoxy, or -NR^{2a}R^{2b} {wherein R^{2a} and R^{2b} may be the same or different and each represents a hydrogen atom, lower alkyl, or -(CH₂)ₘ-Y [wherein m represents an integer of 1 to 6, Y represents hydroxy, lower alkoxy, or -NR^{3a}R^{3b} (wherein R^{3a} and R^{3b} may be the same or different and each represents a hydrogen atom or lower alkyl)], or R^{2a} and R^{2b} are combined together with the adjacent nitrogen atom to form a heterocyclic group }>, or -CH[(CH₂)ₙOH]₂ (wherein n is an integer of 1 to 5)] or a pharmaceutically acceptable salt thereof.

2. The aqueous solution according to claim 1, wherein the pH is 2 to 3;

3. The aqueous solution according to claim 1 or 2, the solution which comprising edetic acid or a salt thereof;

4. The aqueous solution according to claim 3, wherein the content of the edetic acid or the salt thereof is 0.01 to 0.20 weight parts per 1 weight part of the pyrazoloacrydone derivative represented by the general formula (I) or the pharmaceutically acceptable salt thereof.

5. A drug product in which the solution according to any one of claim 1 to 4 is filled into a drug container;

6. A method for stabilizing a pyrazoloacrydone derivative represented by general formula (I): 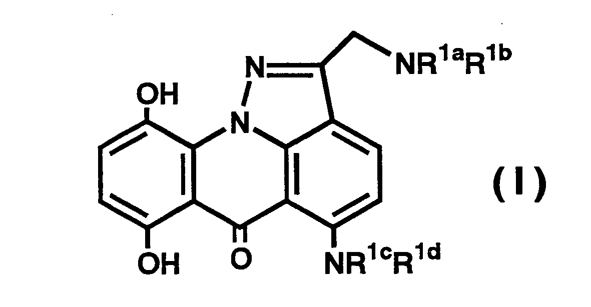 (wherein R^{1a}, R^{1b}, R^{1c}, and R^{1d} have the same meanings as defined above, respectively) or a pharmaceutically acceptable salt thereof in an aqueous solution by adjusting the pH of the aqueous solution comprising the pyrazoloacrydone derivative or the pharmaceutically acceptable salt thereof to 3.5 or less.

7. The method according to claim 6, wherein the pH is adjusted to 2 to 3.
